# EUROPEAN PATENT APPLICATION

(11) **EP 2 959 821 A1**
(43) Date of publication of application: **30.12.2015**
(21) Application number: 14754492.8
(22) Date of filing: 05.02.2014
(51) Int. Cl.: A61B 1/00, G02B 23/26

(54) **ENDOSCOPIC DEVICE**

(30) Priority: 20.02.2013 JP 2013030838
(71) Applicant: OLYMPUS CORPORATION, Shibuya-ku Tokyo 151-0072 (JP)
(72) Inventor: TAKAHASHI, Takeshi, Shibuya-ku Tokyo 151-0072 (JP)
(74) Representative: Schicker, Silvia
(86) International application number: PCT/JP2014/052631
(87) International publication number: WO 2014/129310

(57) **Abstract**

An endoscope includes a heat influence reducing portion that radiates heat, which is generated with light guiding of a light guide portion via a position near a second lens and conducted to the second lens, to a frame member to reduce a local temperature difference in the second lens in a projecting portion in which a first lens of a front observation window through which an inserting direction for insertion into a lumen is observed and the second lens having a truncated conical shape used for observing a viewing field region of a side observation window through which a direction crossing the inserting direction is observed are arranged.

## Description

### BACKGROUND OF THE INVENTION

### 1. Field of the Invention

The present invention relates to an endoscope in which an optical system for front and side observation is arranged at a distal end of an insertion unit.

### 2. Description of the Related Art

In general, there has been known an endoscope having an insertion unit that is inserted into a lumen to observe an image acquired by an imaging unit provided at a distal end portion. An observation optical system provided in the imaging unit is configured with an optical system element (an imaging lens) that can perform not only front observation but also side observation in some cases. For example, in Japanese Patent No. 4955838, a front observation window (a front observation lens) is arranged in a distal end face of a protruding portion provided to protrude from a distal end face of an insertion unit, and a side observation window (a dual purpose lens) having a circular curved surface is arranged on a peripheral surface of the protruding portion.

The front observation window takes in an observation target in a predetermined viewing field region in an inserting direction (an axial direction), and the side observation window takes in an observation target in a side periphery crossing the axial direction. In the observation optical system according to this Japanese Patent No. 4955838, two lenses arranged in the respective windows are integrally configured so that they are aligned in an optical axis direction by a lens frame.

Further, as another example of this observation optical system, in regard to the side observation lens that guides a side observation image taken in from the dual purpose lens which is the side observation window to an imaging element by using a lens surface of the front observation lens as a reflection surface in Japanese Patent No. 4955838, Jpn. Pat. Appln. KOKAI Publication No. 2010-169792 suggests an exit side observation lens that reflects a side observation image taken in from a side observation window on a lens of itself and guides the reflected image to an optical element. In this side observation lens, an exit surface (on a proximal end side) is scraped into a spherical shape, a rear end of the side surface observation window has an acute angle shape extending from a side surface to a back surface, and a side surface has a conical shape having a steps formed due to different diameters.

Furthermore, Jpn. Pat. Appln. KOKAI Publication No. 2001-299677 discloses a countermeasure technology for a problem that heat generated by a light-emitting element (an LED) provided in an insertion unit of an endoscope causes deterioration of quality of a lens or an imaging element.

Specifically, a holding member for the lens or the imaging element is made of a material superior in thermal conductivity, and an outer peripheral surface of the holding member abuts on an outer case having a small wall thickness, thereby efficiently radiating heat.

However, a frame structure of heat conduction is additionally required, miniaturization is difficult, and a complicated structure must be adopted to efficiently radiate heat to the outer case since there is a limit in structure. In particular, since the structure is devised mainly focusing on the front observation window, applying the structure to the side observation window is not easy, and its heat radiation effect is also limited. Further, the number of components in the endoscope is increased, and an assembling operation is complicated, thereby affecting a manufacturing cost.

Thus, it is an object of the present invention to provide an endoscope that avoids a bad influence of heat generated by a heat generator arranged to be adjacent to a photographing optical system lens, has a simple structure in a distal end portion of an insertion unit of a narrow endoscope without degrading quality, has stable performance, and can be realized at a low cost.

### BRIEF SUMMARY OF THE INVENTION

According to an embodiment of the present invention, there is provided an endoscope comprising: a projecting portion having a shape projecting in a tubular shape which is provided at a distal end of an insertion unit inserted into a lumen, and comprises a first observation window which is arranged in a distal end face and formed of a cylindrical first lens used for observing a viewing field region of an inserting direction and a second observation window which is arranged in a peripheral side surface connected to the distal end face and formed of a truncated conical second lens used for observing a viewing field region in a direction crossing the inserting direction; a pedestal which is provided to be adjacent to the protruding portion and in which a first illumination window through which illumination light for illuminating the viewing field region of the first observation window is applied and a light guide portion which guides the illumination light to the first illumination window via a lateral side of the second lens are provided; a frame member which arranges and supports the first lens on a top face of the second lens while overlapping their optical axes; a frame member which fixes a bottom surface side of the second lens and supports a lens group that forms an observation image taken in from the first lens and the second lens; and a heat influence reducing portion which is provided on the bottom surface side of the second lens to abut on the frame member, and reduces concentration of heat, which is generated with light guiding of the light guide portion and conducted to the second lens, on a local part in the second lens.

### BRIEF DESCRIPTION OF THE SEVERAL VIEWS OF THE DRAWINGS

FIG. 1 is a view showing an appearance configuration of an endoscope having an imaging unit provided with a front observation window and a side observation window mounted therein according to a first embodiment;
FIG. 2A is a view showing an appearance configuration of a distal end of an insertion unit according to the first embodiment;
FIG. 2B is a view showing a configuration of the distal end of the insertion unit seen from a front side;
FIG. 3 is a view showing a cross-sectional configuration of a distal end portion including a lens unit taken along a line segment A-A in FIG. 2A;
FIG. 4 is a view showing a cross-sectional configuration of a distal end portion including a lens unit according to a modification of the first embodiment;
FIG. 5A is a view showing a cross-sectional configuration of a distal end portion of an insertion unit according to a second embodiment;
FIG. 5B is a view showing a cross-sectional configuration of a lens unit taken along a line segment B-B in depicted in FIG. 5A;
FIG. 6A is a view showing a cross-sectional configuration of a distal end portion of an insertion unit according to a modification of the second embodiment;
FIG. 6B is a view showing a cross-sectional configuration of a lens unit taken along a line segment B-B depicted in FIG. 6A;
FIG. 7 is a view showing a cross-sectional configuration of a distal end portion of an insertion unit in an endoscope according to a third modification; and
FIG. 8 is a view showing a cross-sectional configuration of a distal end portion of an insertion unit in an endoscope according to a modification of the third embodiment.

### DETAILED DESCRIPTION OF THE PREFERRED EMBODIMENT

An embodiment according to the present invention will now be described hereinafter with reference to the drawings.

### [First Embodiment]

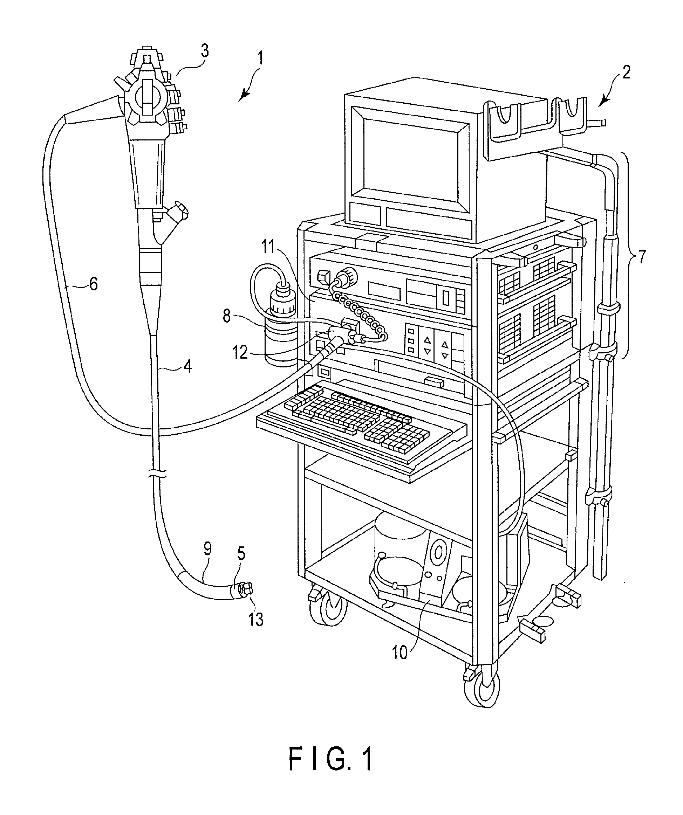

FIG. 1 is a view showing an appearance configuration of an endoscope that has an imaging unit having a front observation window (a direct-viewing observation window) and a side observation window (a side-viewing observation widow) mounted therein according to a first embodiment.

The endoscope according to this embodiment is roughly constituted of an endoscope main body 1 and an endoscope device 7 mounted in a movable trolley 2. This embodiment can be applied to a biological endoscope for observing the inside of a body cavity or the inside of a lumen of a living matter or an industrial endoscope for observing the inside of a device such as an engine or the inside of a pipe line. Further, in this embodiment, a flexible scope will be taken as an example and described, but this embodiment can be likewise mounted in a rigid scope.

The endoscope main body 1 is constituted of an insertion unit (a flexible tune) 4 that is inserted into a lumen that is an observation target, a bending unit 9 provided at a distal end of the insertion unit 4, and an operation unit 3 that operates the bending unit 9 to bend. A distal end portion 5 where an imaging unit or an illumination unit is arranged is provided on a distal end side of the bending unit 9. In the following description, the insertion unit 4 is determined as a center, a side closer to the distal end portion 5 will be referred to as distal end side, and a side closer to the operation unit 3 will be referred to as a proximal end side.

The endoscope device 7 has a light source device that generates illumination light applied to an observation target region, a video processor that executes predetermined image processing to an acquired video signal, a monitor that displays the video signal as an observation image, a keyboard as an input unit, and others.

Moreover, a bottle 8 that stores a liquid used for cleaning or the like (a cleaning liquid: e.g., a liquid mainly containing water such as a physiological saline solution) is detachably disposed to a main column of the trolley 2. Additionally, an air supply pump unit is arranged in the endoscope device 7. Further, a suction unit 10 that sucks a liquid or a gas for cleaning that has been injected into a lumen from a later-described cleaning nozzle in the lumen is provided on a rack of the trolley 2.

The endoscope main body 1 and a light source unit 11 are connected to a universal cable 6 through a connector. The universal cable 6 includes not only a light guide formed of an optical fiber but also signal lines through which a video signal and others are transmitted, and a supply path (an air supply/liquid supply channel) and a discharge path for a gas and a liquid that are formed of tubes. A connector portion 12 connected to the endoscope device 7 side of the universal cable 6 is branched relative to the signal lines, the tubes, and the light guide and connected to respective constituent portions.

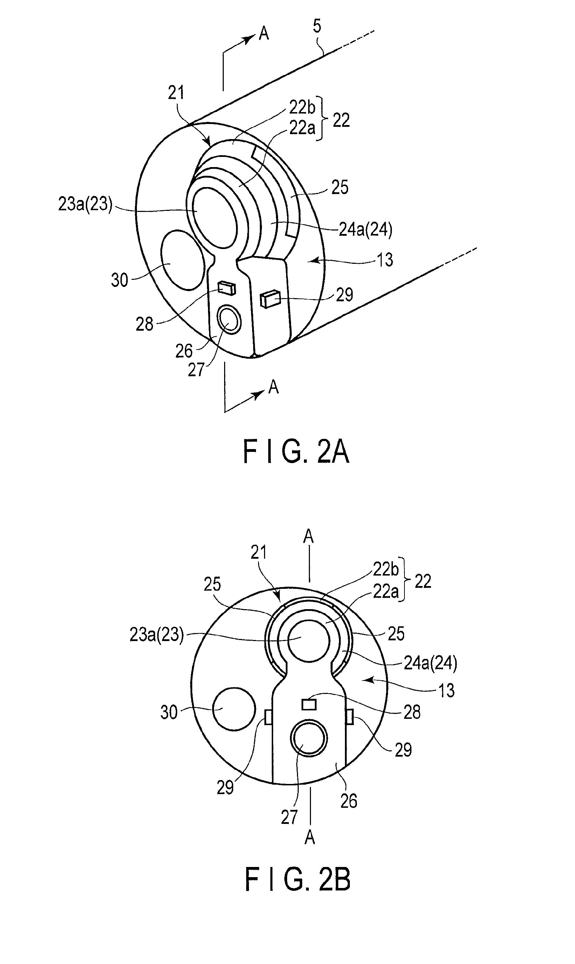

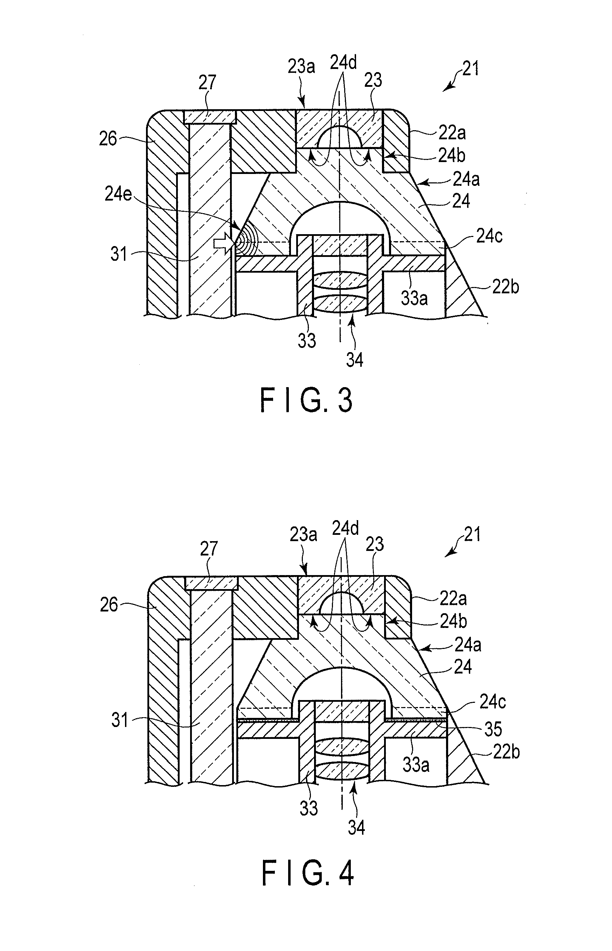

FIG. 2A is a view showing an appearance configuration of a distal end of the insertion unit according to the first embodiment, and FIG. 2B is a view showing a configuration of the distal end of the insertion unit seen from the front side. FIG. 3 is a view showing a cross-sectional configuration of the distal end portion 5 including a lens unit 21 taken along a line section A-A in FIG. 2A. It is to be noted that a direction along which the insertion unit travels in a body cavity will be referred to as an inserting direction or an axial direction, a surface seen from the axial direction will be referred to as a front surface (a distal end face), and a surface orthogonal to the axial direction will be referred to as a side surface or a peripheral side surface.

A protruding portion (a pedestal) 13 that has the lens unit 21 and the a pedestal 26 integrally provided thereto and protrudes in a tubular form is provided on the distal end face of the distal end portion 5 of the insertion unit 4. The lens unit 21 is part of an imaging optical system of the endoscope, and it is constituted of a front observation window (a first observation window: a cylindrical concave lens 23 [a first lens]) 23a for observing a predetermined viewing field region (a first viewing field region) in the inserting direction (the axial direction), a side observation window (a second observation window: a truncated conical lens 24 [a second lens]) 24a for observing a predetermined viewing field region in a direction (a lateral side) crossing the inserting direction, and a lens group 34 that leads an optical image of an observation image condensed by the truncated conical lens 24 to a non-illustrated imaging element. It is to be noted that, in this embodiment, a surface of the cylindrical concave lens 23 is the front observation window 23a and a tapered conical surface (or a side surface) of the truncated conical lens 24 is the side observation window 24a. Two side illumination windows (second illumination windows) 25 used for illuminating entire circumferences on lateral sides are arranged at the rear of the lens unit 21.

Moreover, the pedestal 26 is a distal end structure having the same surface height (a height of projection toward the front side) as the front observation window 23a. Additionally, a cleaning nozzle 28 arranged near the front observation window 23a and a front illumination window (a first illumination window) 27 that applies illumination light for front observation are arranged on a front surface side of the pedestal 26. Further, two cleaning nozzles 29 used for cleaning the side observation window 24a are arranged on both side surfaces of the pedestal 26. A liquid supply path and an air supply path, which are not shown, connected to the cleaning nozzles 28 and 29 are arranged in the pedestal 13, and a switching valve is provided in the middle of the pipe arrangement. Furthermore, an opening portion 30 as a forceps hole through which a non-illustrated forceps or the like is inserted is formed in the distal end face near the lens unit 21.

Moreover, a light guide (a light guide portion) 31, which is inserted from a proximal end of the insertion unit to be adjacent to the lens unit 21 in the protruding portion, is arranged to reach the front illumination winnow 27 arranged on the distal end face of the pedestal 26 and guides illumination light (a light flux). The guided illumination light is applied toward the front direction to illuminate an observation viewing field of the front observation window 23a. The light guide 31 and the front illumination window 27 serve as heat generation sources at the time of illumination as described above. It is to be noted that, in this embodiment, the configuration using the light guide 31 is provided, but the present invention can be likewise applied to heat generation effected by a light source other than the light guide 31 or a heat source (e.g., a heater), e.g., a configuration where a light-emitting element such as a light-emitting diode is provided on the distal end side of the insertion unit.

The lens unit 21 will now be described in detail.

As shown in FIG. 3, the cylindrical concave lens 23 arranged on the front surface has a cylindrical shape, its front surface side (an incidence side) exposed to the front side has a flat surface, and a semispherical concave curved surface is formed at the center of a back surface side (an exit side) that abuts on the truncated conical lens 24.

A periphery of a top surface of the truncated conical lens 24 (a surface on a small-diameter side) is cut off in an annular shape, and a step portion 24b having the same diameter as the cylindrical concave lens 23 is formed. The cylindrical concave lens 23 is arranged to abut on the top face of the truncated conical lens 24 so that their optical axes overlap, and these members are integrally supported by a lens frame 22a.

Additionally, a semispherical concave surface is formed at the center of a bottom surface (a surface on a large-diameter side) side of the truncated conical lens 24, and a heat influence reducing portion 24c projecting in a ring-like shape is formed around the concave surface. This heat influence reducing portion 24c is a peripheral side surface whose outer peripheral surface connected with the side observation window 24a forming at least a conical surface is a vertical. That is, the bottom surface side of the truncated conical lens 24 is annularly projected with a uniform thickness around the optical axis so that a conventional acute angle shape is changed into an obtuse angle shape. Further, the truncated conical lens 24 is fitted into the lens frame 22b except a portion abutting on the pedestal 26 by using the peripheral side surface of the heat influence reducing portion 24c. Furthermore, the truncated conical lens 24 (the heat influence reducing portion 24c) has its bottom surface fitted to a frame member 33 that supports the lens group 34, and it is fixed in an abutting or close contact manner.

An abutting surface 24d of the truncated conical lens 24 and the cylindrical concave lens 23 forms a reflection surface of the truncated conical lens 24. The truncated conical lens 24 is supported by the frame member 33 in the pedestal 13.

In the frame member 33, a tubular portion serving as a light path is arranged at the center, and a flange-like projecting portion 33a is formed at a distal end and appressed against the bottom surface of the truncated conical lens 24. The lens group 24 formed of image forming lenses is fitted in the tubular portion so that it is aligned on the optical axis.

A relationship between such a lens unit 21 and heat generated by the light guide 31 will now be described.

As described above, the truncated conical lens 24 of the lens unit 21 is arranged in proximity to the light guide 31. Therefore, as shown in FIG. 3, heat generated by the light guide 31 is conducted a portion 24e of the heat influence reducing portion 24c of the truncated conical lens 24 which is closest. On the other hand, a portion on the opposite side in the radial direction has a temperature that is substantially equal to an ambient temperature, and a temperature difference is produced in the truncated conical lens 24 at the beginning.

A specific example of the temperature difference produced in the truncated conical lens 24 due to heat will now be described. If the heat influence reducing portion 24c is not formed on the truncated conical lens 24, heat is prone to be stored in an angular portion having an acute angle that is connected with the bottom portion (the proximal end face) on the lens lower side, and a temperature rises to a value close a temperature of the light guide that increases by the illumination light. For example, a temperature near the adjoining angular portion is assumed to increase to approximately 80°C. A temperature of the truncated conical lens 24 is lowered as distanced from this angular portion, and it becomes a temperature closer to an outside air temperature at the portion on the opposite side.

That is, a temperature difference between 80°C and 20°C is generated in the same lens. When this temperature difference is multiplied by a general glass linear thermal expansion coefficient (e.g., 7×10⁻⁶/°C), 0.42 µm can be obtained. That is, a large error difference, which is 0.42 µm, is generated in the same lens. This value becomes a large error with respect to an optical surface shape error (a requested accuracy) of the lens and results in image deterioration such as an aberration.

The truncated conical lens 24 diffuses the heat propagated from the light guide 31 in the lens and radiates it through the frame member 33 and the lens frame 22a. In this embodiment, when the heat influence reducing portion 24c is formed on the lower portion of the truncated conical lens 24, to which heat is conducted most, to provide the obtuse angle shape, concentration of heat can be alleviated, a difference of heat in the lens can be reduced, and deformation (a distortion) or crack damage due to heat can be avoided. Further, when the distortion produced due to a local temperature difference in the lens is suppressed, an observation image can pass through a designed light path, and image quality of an acquired image can be prevented from lowering.

### [Modification of First Embodiment]

A modification of the first embodiment will now be described with reference to FIG. 4.

FIG. 4 is a view showing a cross-sectional configuration of the distal end of the insertion unit including the lens unit taken along a line segment A-A in FIG. 2A. In this modification, a technique of fixing the truncated conical shape 24 of the lens unit 21 is different, other constituent part as well as the endoscope system are the same except for these members, and like reference numerals are provided to omit a description thereof.

The cylindrical concave lens 23 is arranged to be continuous with the truncated conical shape 24 in such a manner that their optical axes can be matched with each other. A semispherical concave surface is formed at the center of the lower surface side of the truncated conical lens 24, and the heat influence reducing portion 24c projecting in a ring-like shape is formed around the concave surface. In this heat influence reducing portion 24c, at least its outer peripheral surface is formed as a vertical surface, and a bottom surface of the heat influence reducing portion 24c is fixed to the flange-like projecting portion 33a of the frame member 33 with the use of an adhesive. For example, when an epoxy-based adhesive material is used as the adhesive, hardening shrinkage of the adhesive due to heat occurs, and the surface of the lens is contracted.

On the other hand, in this modification, since the heat influence reducing portion 24c is provided on the lower side of the truncated conical lens 24 and the bottom surface (a bonding surface) that is affected by heat most and the optical function surface (the side observation window 24a) are separated from each other, an influence of shrinkage of the adhesive is hardly exerted, and an excellent image can be provided.

### [Second Embodiment]

A heat influence reducing portion according to a second embodiment will now be described.

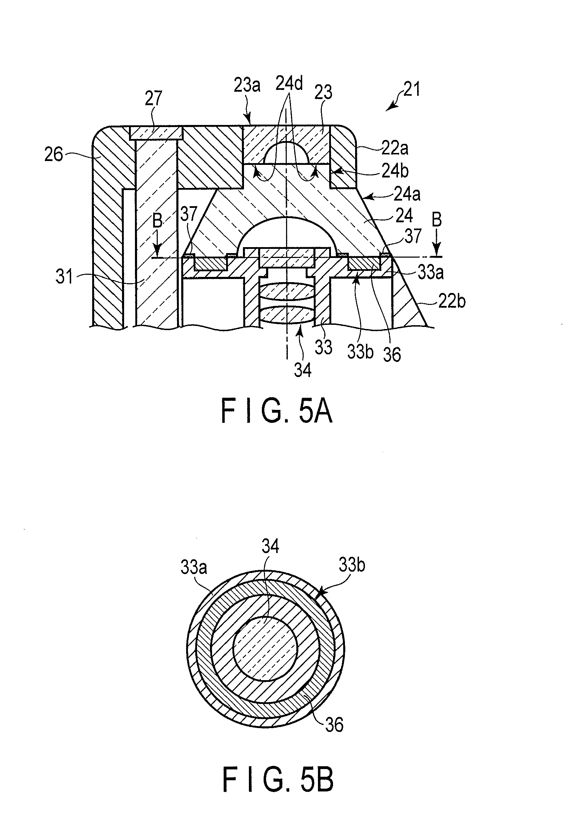

FIG. 5A is a view showing a cross-sectional configuration of a distal end portion of an insertion unit in an endoscope according to a second embodiment, and FIG. 5B is a view showing a cross-sectional configuration of a lens unit 21 taken along a line segment B-B depicted in FIG. 5A. That is, the cross-sectional configuration of the distal end portion taken along the line segment A-A in FIG. 2A described in the first embodiment is shown. It is to be noted that, in constituent parts in this embodiment, like reference numerals denote constituent parts equal to the constituent parts in the first embodiment to omit a detailed description thereof.

The lens unit 21 in this embodiment has a configuration that a heat conduction member (a gel-like material, a paste-like material, or a sheet-like material) 35 that is a heat influence reducing portion annularly provided in a flange-like projecting portion 33a provided at a distal end of a frame member 33 is arranged to abut on or to be appressed against a bottom surface of a truncated conical lens 24 of a side observation window 24a and a heat radiation effect of the truncated conical lens 24 is thereby enhanced.

As shown in FIG. 5A and 5B, an annular groove 33b is formed at a portion of the projecting portion 33a to which the bottom surface of the truncated conical lens 24 is fixed. The inside of the annular groove 33b is filled with a heat conduction portion 36, the bottom surface portion of the truncated conical lens 24 excluding the groove is bonded and fixed by an adhesive 37 and sealed so that the heat conduction portion 36 does not protrudes. At this time, the heat conduction member 35 must abut on or must be appressed against the bottom surface of the truncated conical lens 24. It is to be noted that the heat conduction member is not restricted to a gel state or the like, and it may be formed into a sheet-like shape or may be fitted in the groove.

The heat conduction portion 36 has high thermal conductivity and, for example, one containing silicone as a main starting material is known. When a later-described heat conductive adhesive is adopted as the adhesive 37, heat can be released to a lens, a frame member 33, or a lens frame 22 through bonding surfaces of the bottom surface of the truncated conical lens 24 and the projecting portion 33a.

The truncated conical lens 24 in the thus configured lens unit 21 releases the heat, which has been conducted from a light guide 31, to the annular heat conduction portion 36 from the bottom surface of the lens, thereby efficiently radiating the heat through the frame member 33 or the lens frame 22.

Therefore, according to this embodiment, the heat can be conducted to a portion having a temperature close to a room temperature in the truncated conical lens 24, i.e., the opposite side of the side adjacent to the light guide 31 through the heat conduction portion 36, the heat on the adjacent side can be diffused to the opposite side, and releasing the stored heat enables alleviating a local temperature difference in the truncated conical lens 24, thereby increasing a temperature of the entire lens. Therefore, when a distortion caused due to a temperature difference in the lens is suppressed, image quality of an acquired image can be prevented from lowering.

### [Modification of Second Embodiment]

A modification of the second embodiment will now be described.

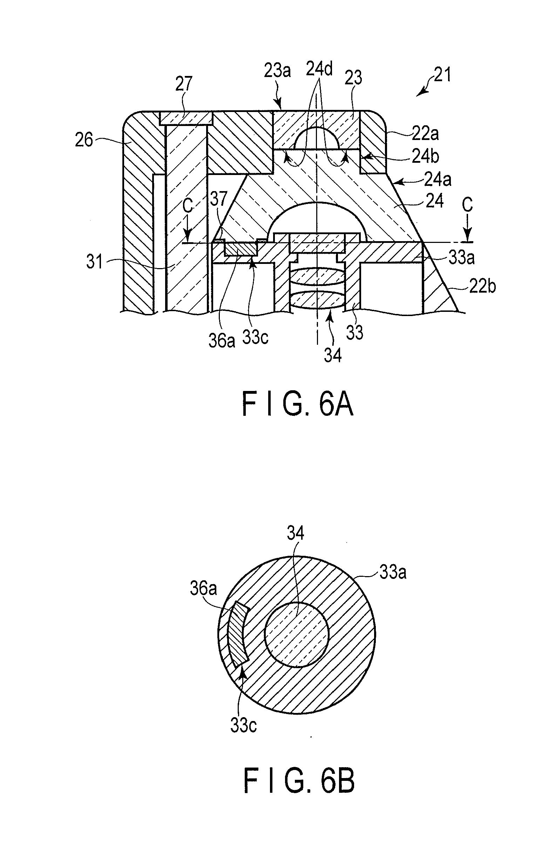

FIG. 6A is a view showing a cross-sectional configuration of the distal end portion of the insertion unit in the endoscope according to a modification of the second embodiment, and FIG. 6B is a view showing a cross-sectional configuration of the lens unit 21 taken along a line segment B-B depicted in FIG. 6A. It is to be noted that, in constituent parts according to this embodiment, like reference numerals denote constituent parts equal to the constituent parts in the first embodiment to omit a detailed description thereof.

This modification has a configuration that an arc groove 33c is formed at a position on the projecting portion 33a close to the light guide 31 in the second embodiment and an arc-shaped heat conduction portion 36a filled with a heat conduction material is arranged.

This modification can provide the same functions and effects as those of the second embodiment. Moreover, when a later-described heat conductive adhesive is adopted as the adhesive, heat can be further released to the lens, the frame member 33, or the lens frame 22 through bonding surfaces of the bottom surface of the truncated conical lens 24 and the projecting portion 33a. That is, even if a local temperature increase at an angular portion on the lower side of the truncated conical lens 24 close to the light guide 31 is suppressed and heat is conducted, the heat can be diffused in the lens. Therefore, when a distortion caused due to a local temperature difference (concentration of the heat) in the lens is suppressed, an observation image can pass through a light path in the designed lens, and image quality of an acquired image can be prevented from lowering.

### [Third Embodiment]

A heat influence reducing portion according to a third embodiment will now be described.

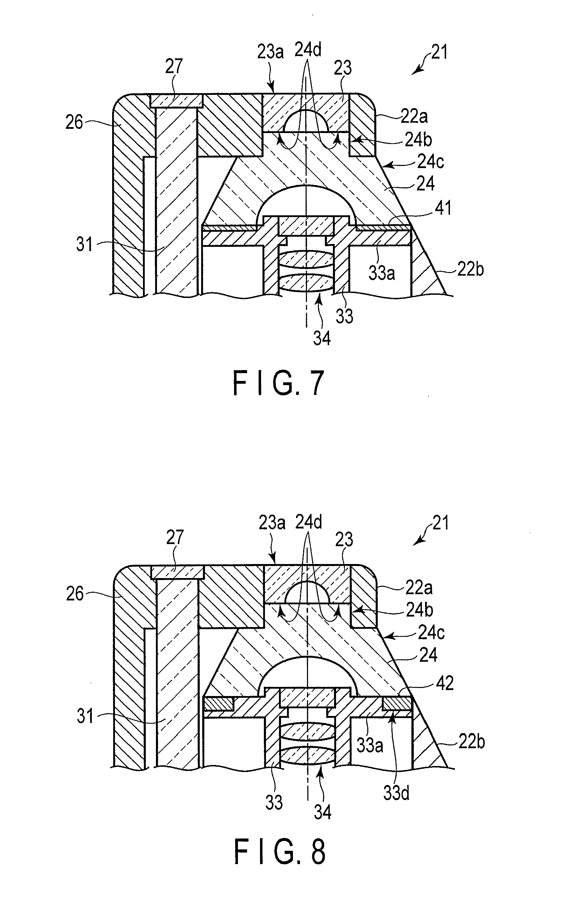

FIG. 7 is a view showing a cross-sectional configuration of a distal end portion of an insertion unit in an endoscope according to a third embodiment.

This embodiment has a configuration in which an overall bottom surface of a truncated conical lens 24 is bonded to a projecting portion 33a through a heat conductive adhesive having high thermal conductivity and heat is released to the lens, a frame member 33, or a lens frame 22 through the adhesive.
As an adhesive 41, this embodiment adopts the heat conductive adhesive that functions as a heat influence reducing portion. There is known an adhesive using a resin material (e.g., an epoxy material, a polyimide material, a silicone material, and others) as the heat conductive adhesive 41.

According to this embodiment, since the heat conducive adhesive 41 is used, conducting the heat to a position in the truncated conical lens 24 having a temperature close to a room temperature, i.e., the opposite side of the side close to a light guide 31 enables diffusing the heat on the adjacent side to the opposite side, and a local temperature difference (concentration of the heat) in the truncated conical lens 24 is alleviated. Therefore, when a distortion produced due to a temperature difference in the lens is suppressed, image quality of an acquired image can be prevented from lowering. It is to be noted that the description has been given as to the example where the heat is diffused by interposing the heat conductive adhesive 41, but the same functions and effects can be provided when a heat conductive sheet is sandwiched between bonding surfaces of the truncated conical lens 24 and the projecting portion 33a in place of the adhesive.

### [Modification of Third Embodiment]

A modification of the third embodiment will now be described.

FIG. 8 is a view showing a cross-sectional configuration of the distal end portion of the insertion unit in the endoscope according to a modification of the third embodiment. Although the third embodiment has the configuration where the overall bottom surface of the truncated conical lens 24 is bonded to the projecting portion 33a through the heat conductive adhesive, this modification has a configuration where a bottom surface of the truncated conical lens 24 on an outer peripheral side is bonded to the projecting portion 33a through a heat conductive adhesive that functions as the heat influence reducing portion.

As shown in FIG. 8, the outer peripheral side of the surface of the projecting portion 33a is scraped away to form a step 33d. The heat conductive adhesive is put to fill this step 33d, and the outer peripheral side portion of the bottom surface of the truncated conical lens 24 is bonded to the projecting portion 33a.

In this modification, likewise, heat conducted to the truncated conical lens 24 can be released to the opposite side of the lens (the opposite side of a light guide 31), a frame member 33, or a lens frame 22 through the heat conductive adhesive 42. This heat release can ease a local temperature difference (concentration of the heat) in the truncated conical lens 24, suppress a distortion produced due to a temperature difference in the lens, and prevent image quality of an acquired image from lowering.

The embodiment according to the present invention has the following technical features.
(1) An endoscope having a side observation optical system for observing a viewing field including a direction substantially orthogonal to a longitudinal direction of the insertion unit on a distal end side of the insertion unit that is inserted into a lumen, comprising:
   an annular objective lens whose outer diameter differs depending on a position closer to a distal end of the insertion unit and a position closer to a proximal end of the same and which is provided in the side observation optical system;
   a light guide portion that is adjacent to the objective lens and guides illumination light that is required for the observation with respect to a viewing field of the endoscope; and
   a heat influence reducing portion that is provided in contact with one end portion side of the insertion unit in the longitudinal direction in the objective lens and reduces an influence of heat applied to the objective lens from the light guide portion on the objective lens.
(2) The endoscope according to Section (1),
   wherein the heat influence reducing portion is a distortion inhibiting portion that inhibits the objective lens from being thermally deformed.
(3) The endoscope according to Section (1),
   wherein the heat influence reducing portion is provided on a side where the objective lens has a larger diameter.
(4) The endoscope according to Section (1),
   wherein the heat influence reducing portion is provided to be integrally contact with the one end portion of the objective lens.
(5) The endoscope according to Section (4),
   wherein the heat influence reducing portion is integrally formed to have a surface that is continuous with the one end portion of the objective lens.
(6) The endoscope according to Section (5),
   wherein the objective lens has optical characteristics also in the annular inner surface, and
   the heat influence reducing portion is integrally formed to have the optical characteristics continuously with the inner surface of the objective lens.
(7) The endoscope according to Section (1),
   wherein the heat influence reducing portion has a cross section including a circular shape like the one end portion of the objective lens.
(8) The endoscope according to Section (1),
   wherein the heat influence reducing portion is provided on a side which faces the light guide portion at the one end portion of the objective lens.
(9) The endoscope according to Section (1),
   wherein a lens frame which adheres to the objective lens and the holds the objective lens is provided on the one end portion side of the objective lens, and
   the heat influence reducing portion also functions as a distortion reducing portion that buffers distortion produced in a bonded portion of the objective lens and the lens frame.
(10) The endoscope according to Section (1), further comprising a front observation optical system for observing a viewing field including a direction parallel to the longitudinal direction of the insertion unit on the distal end side of the insertion unit that is inserted into a lumen.

## Claims

1. An endoscope comprising:
a projecting portion having a shape projecting in a tubular shape which is provided at a distal end of an insertion unit inserted into a lumen, and comprises a first observation window which is arranged in a distal end face and formed of a cylindrical first lens used for observing a viewing field region of an inserting direction and a second observation window which is arranged in a peripheral side surface connected to the distal end face and formed of a truncated conical second lens used for observing a viewing field region in a direction crossing the inserting direction;
a pedestal which is provided to be adjacent to the protruding portion and in which a first illumination window through which illumination light for illuminating the viewing field region of the first observation window is applied and a light guide portion which guides the illumination light to the first illumination window via a lateral side of the second lens are provided;
a frame member which arranges and supports the first lens on a top face of the second lens while overlapping their optical axes;
a frame member which fixes a bottom surface side of the second lens and supports a lens group that forms an observation image taken in from the first lens and the second lens; and
a heat influence reducing portion which is provided on the bottom surface side of the second lens to abut on the frame member, and reduces concentration of heat, which is generated with light guiding of the light guide portion and conducted to the second lens, on a local part in the second lens.

2. The endoscope according to claim 1,
wherein the heat influence reducing portion is provided on a bottom surface of the second lens to project in a ring-like shape with a vertical outer peripheral surface that surrounds the optical axis.

3. The endoscope according to claim 1,
wherein the heat influence reducing portion is a heat conduction member formed of one of gel-like, paste-like, and sheet-like heat conduction members that is put in an annular groove formed in the frame member and abuts on a bottom surface of a conical surface of the second lens.

4. The endoscope according to claim 1,
wherein the heat influence reducing portion is a heat conduction member made of one of a gel-like material and a paste-like material that is put in an arc groove formed in the frame member on a side closer to the light guide portion and abuts on a bottom surface of a conical surface of the second lens.

5. The endoscope according to claim 1,
wherein the heat influence reducing portion is a heat conductive adhesive that enables an overall bottom surface or a bottom surface on at least an outer peripheral side of the second lens to adhere to the frame member.
